(19) Europäisches Patentamt

European Patent Office

Office européen des brevets

(11) Veröffentlichungsnummer : **0 341 558 B1**

## (12) EUROPÄISCHE PATENTSCHRIFT

(45) Veröffentlichungstag der Patentschrift :
**29.07.92 Patentblatt 92/31**

(21) Anmeldenummer : **89107969.1**

(22) Anmeldetag : **03.05.89**

(51) Int. Cl.⁵ : **A61K 37/02,** // (A61K37/02, 31:55)

(30) Priorität : **11.05.88 DE 3816169**

(43) Veröffentlichungstag der Anmeldung :
**15.11.89 Patentblatt 89/46**

(45) Bekanntmachung des Hinweises auf die
Patenterteilung :
**29.07.92 Patentblatt 92/31**

(84) Benannte Vertragsstaaten :
**AT BE CH DE ES FR GB GR IT LI LU NL SE**

(56) Entgegenhaltungen :
**WO-A-88/02632**

(56) Entgegenhaltungen :
**NATURE, Band 330, Nr. 17, Dezember 1987,
Seiten 662-664, London; K.J. TRACEY et al.:
"Anti-cachectin/TNF monoclonalantibodies
prevent septic shock during lethal bacteraemia"**

(73) Patentinhaber : **BOEHRINGER INGELHEIM KG
W-6507 Ingelheim am Rhein (DE)**
㊴ **BE CH DE ES FR GR IT LI LU NL SE AT**
Patentinhaber : **BOEHRINGER INGELHEIM
INTERNATIONAL G.M.B.H.
W-6507 Ingelheim am Rhein (DE)**
㊴ **GB**

(72) Erfinder : **Heuer, Hubert, Dr.
Am Alten Weg 29
W-6500 Mainz 32 (DE)**

(54) **Verwendung und Mittel zur Verminderung der Nebenwirkungen von TNF.**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 341 558 B1

## Beschreibung

Die Erfindung betrifft die Verwendung von PAF-Antagonisten zur Verminderung unerwünschter Nebenwirkungen, die bei der therapeutischen Anwendung von TNF auftreten.

Tumor-Nekrose-Faktoren sind Proteine, die primär von Monozyten/Makrophagen (TNF-$\alpha$) bzw. Lymphozyten (TNF-ß), aber auch von anderen Zelltypen des Organismus nach entsprechender Stimulation gebildet werden. Diese Proteine zeigen cytostatische und cytotoxische Wirkung auf Tumorzellen in vitro und therapeutische Wirkung in verschiedenen in vivo - Tumormodellen in Versuchstieren. Darüber hinaus zeigen TNFs antivirale Aktivität gegen verschiedene Virustypen, aktivieren die Immunabwehr gegen Parasiten, und wirken direkt und/oder indirekt als Mediatoren bei Immunreaktionen, entzündlichen Prozessen und anderen Vorgängen im Organismus, wobei die Wirkungsmechanismen in vielen Fällen noch ungeklärt sind.

Eine Reihe von Beobachtungen läßt auf eine Rolle von endogen freigesetzten TNFs bei verschiedenen pathologischen Zuständen schließen. So scheint TNF-$\alpha$ ein Mediator der Kachexie zu sein, die bei chronisch-invasiven Erkrankungen auftreten kann. TNF-$\alpha$scheint auch eine wesentliche Rolle bei der Pathogenese des durch gram-negative Bakterien verursachten Schocks (Endotoxin-Schock) zu spielen. Ebenso wurde eine Funktion von TNF bei den im Rahmen von entzündlichen Prozessen in Gelenken und anderen Geweben auftretenden Gewebeschädigungen postuliert.

Aus diesen Erkenntnissen ergeben sich einerseits mögliche therapeutische Anwendungen der TNFs in der Onkologie, Virologie, Parasitologie und bei bakteriellen Infektionen; andererseits ist die therapeutische Anwendung jedoch durch dosisabhängige Nebenwirkungen, insbesondere durch die schock-induzierende Wirkung der TNFs, limitiert. Wie oben beschrieben, kann auch die endogene Freisetzung von TNFs unerwünschte Wirkungen zeigen.

Es wurde nun gefunden, daß überraschenderweise durch gleichzeitige Gabe von PAF-Antagonisten die unerwünschten Nebenwirkungen von TNFs, z.B. schockähnliche Zustände, verringert und die schockinduzierte Verminderung der gastrointestinalen Passagegeschwindigkeit gehemmt werden kann.

Im Tierexperiment konnte auch die durch TNF-Gabe induzierte Mortalität von Mäusen dosisabhängig durch PAF-Antagonisten (z.B. Web 2347) wie auch das Priming (Prägung) durch TNF für eine erhöhte Sensivität von Mäusen auf eine zweite Gabe von TNF überraschenderweise in vivo gehemmt werden.

Unter "gleichzeitiger Gabe" ist die Verabreichung von TNFs und PAF-Antagonist in einer gemeinsamen Formulierung oder in zeitlichem Zusammenhang in getrennten Formulierungen zu verstehen. Beispielsweise kann der PAF-Antagonist vor der Anwendung des TNF appliziert werden, wobei das Zeitintervall von der Wirkungsdauer des PAF-Antagonisten mitbestimmt wird, d.h. daß TNF appliziert wird, solange die Wirkung des PAF-Antagonisten noch ausreichend stark ist. Andererseits kann der PAF-Antagonist auch kurze Zeit nach der TNF Gabe verabreicht werden.

PAF-Antagonisten sind in großer Zahl beschrieben worden, beispielsweise in den Europa-Anmeldungen 176 927, 176 928, 176 929, 194 416, 230 942, 240 899, 254 245, 255 028, auf deren Inhalt Bezug genommen wird.

Die in diesen Veröffentlichungen offenbarten PAF-Antagonisten eignen sich für die Anwendung gemäß der Erfindung. Hervorzuheben ist die Verwendung von 4-(2-Chlorphenyl)-9-methyl-2-[3-(4-morpholinyl)-3-propanon-1-yl]-6H-thieno[3,2-f][1,2,4]triazolo[4,3-a][1,4]diazepin (WEB 2086); 6-(2-Chlorphenyl)-8,9-dihydro-1-methyl-8-[(4-morpholinyl)carbonyl]-4H,7H-cyclopenta[4,5]thieno[3,2-f][1,2,4]triazolo[4,3-a][1,4]diazepin (Web 2170) oder das 6-(2-Chlorphenyl)-8,9-dihydro-1-methyl-8-[dipropylaminocarbonyl]-4H,7H-cyclopenta[4,5]thieno[3,2-f][1,2,4]triazolo[4,3-a][1,4]diazepin Web 2347) bzw. deren Säureadditionssalze sowie der in den oben genannten Europaanmeldungen hervorgehobenen Verbindungen.

Die Wirkstoffe werden in Form üblicher galenischer Zubereitungen angewendet. Für TNFs eignen sich im wesentlichen Injektionslösungen, so daß auch gemeinsame Formulierungen mit PAF-Antagonisten im allgemeinen die Form von Injektionslösungen haben. Werden die Wirkstoffe in getrennten Formulierungen verabreicht, können die PAF-Antagonisten in den für sie geeigneten literaturbekannten Zubereitungsformen angewendet werden.

Die Dosierung liegt im allgemeinen im Rahmen der für die Einzelwirkstoffe in der Literatur vorgeschlagenen Dosierung. Da jedoch durch die kombinierte Anwendung die therapeutische Breite für TNFs vergrößert wird, kann TNF gewünschtenfalls auch in höherer Dosis verabreicht werden, als es bei der alleinigen Anwendung vertretbar wäre.

## Patentansprüche

1. Verwendung von PAF-Antagonisten zur Herstellung eines Arzneimittels zur Verminderung der unerwünschten Nebenwirkungen bei der therapeutischen Anwendung von TNF.

2. Verwendung nach Anspruch 1, dadurch gekennzeichnet, daß der PAF-Antagonist web 2086, web 2170 oder web 2347 gegebenenfalls in Form eines Säureadditions Salzes ist.

3. Injectionslösung, gekennzeichnet durch einen Gehalt an TNF und einen PAF-Antagonisten.

4. Injectionslösung nach Anspruch 3, dadurch gekennzeichnet, daß der PAF-Antagonist Web 2086, Web 2170 oder Web 2347 gegebenenfalls in Form eines Säureadditionssalzes, ist.

## Claims

1. Use of PAF-antagonists for preparing a pharmaceutical composition for reducing the undesirable side effects in the therapeutic use of TNF.

2. Use according to claim 1, characterised in that the PAF-antagonist is Web 2086, Web 2170 or Web 2347, optionally in the form of an acid addition salt.

3. Injectable solution, characterised in that it contains TNF and a PAF-antagonist.

4. Injectable solution according to claim 3, characterised in that the PAF-antagonist is Web 2086, Web 2170 or Web 2347, optionally in the form of an acid addition salt.

## Revendications

1. Utilisation d'antagonistes du PAF pour la préparation d'un médicament pour réduire les effets secondaires indésirables lors de l'utilisation thérapeutique de TNF.

2. Utilisation selon la revendication 1, caractérisée en ce que l'antagoniste du PAF est Web 2086, Web 2170 ou Web 2347, éventuellement sous forme d'un sel d'addition d'acide.

3. Solution pour injection, caractérisée par une teneur en TNF et en un antagoniste du PAF.

4. Solution pour injection selon la revendication 3, caractérisée en ce que l'antagoniste du PAF est Web 2086, Web 2170 ou Web 2347, éventuellement sous forme d'un sel d'addition d'acide.